# EUROPEAN PATENT SPECIFICATION

(11) **EP 3 212 608 B1**
(45) Date of publication and mention of the grant of the patent: **30.12.2020**
(21) Application number: 15791386.4
(22) Date of filing: 07.10.2015
(51) Int. Cl.: C07C 67/39, C07C 69/54, C07C 45/37

(54) **OXIDATIVE ESTERIFICATION PROCESS FOR MAKING METHYL METHACRYLATE**
OXIDATIVES VERESTERUNGSVERFAHREN ZUR HERSTELLUNG VON METHYLMETHACRYLAT
PROCÉDÉ D'ESTÉRIFICATION OXYDATIVE POUR LA FABRICATION DE MÉTHACRYLATE DE MÉTHYLE

(30) Priority: 31.10.2014 US 201462073290 P
(43) Date of publication of application: 06.09.2017
(73) Proprietor: Rohm and Haas Company, Philadelphia, PA 19106 (US)
(72) Inventor: LIMBACH, Kirk, W., Dresher, PA 19025 (US); KRAPTCHETOV, Dmitri, A., Lansdale, PA 19446 (US); FRICK, Christopher, D., Pottstown, PA 19465 (US)
(74) Representative: Murgitroyd & Company
(86) International application number: PCT/US2015/054437
(87) International publication number: WO 2016/069225

(56) References cited:
- EP-A1- 0 972 759
- GB-A- 2 094 782
- US-A- 5 892 102
- US-A- 6 040 472
- DATABASE WPI Week 199834 Thomson Scientific, London, GB; AN 1998-393427 XP002752936, & JP H10 158214 A (MITSUBISHI RAYON CO LTD) 16 June 1998 (1998-06-16)

## Description

### BACKGROUND OF THE INVENTION

The invention relates to a process for the preparation of carboxylic acid esters via oxidative esterification.

The production of methyl methacrylate (MMA) from methacrolein (MAL), methanol, and oxygen is known. For example, US 4,518,796 discloses the use of a Pd - bismuth (Bi) catalyst. However, that catalyst did not give high MMA selectivity, which is desired for this reaction.

US 5,892,102 discloses MAL oxidative esterification catalysts that include Pd-Bi-X intermetallics, where X can be a variety of elements, on a ZnO or CaCO₃ support. These supports are undesirable from a mechanical stability, likely acid resistance, and long-term catalyst life standpoint.

EP0972759A1 discloses a process for producing a carboxylic acid ester, comprising reacting an aldehyde and an alcohol in a liquid phase in the presence of molecular oxygen by the use of a catalyst comprising at least palladium and X, X representing bismuth and/or lead, supported on a carrier, wherein the catalyst used has an acid strength, pKa, of more than 4.8 and shows an ammonia chemical adsorption amount at 0 °C of 0 - 150 µmol/g-catalyst.

US5892102A discloses a catalyst for the production of carboxylic acid esters for use in reacting an aldehyde with an alcohol in a liquid phase in the presence of molecular oxygen, comprising calcium carbonate and palladium, bismuth and at least one element selected from the group consisting of barium, iron, zinc and germanium, these elements being supported on said calcium carbonate.

JPH10158214A discloses a process for producing a carboxylate by reacting an aldehyde with an alcohol in liquid phase in the presence of molecular oxygen using a catalyst containing palladium, X and Y (X is bismuth and/or lead; Y is at least one element selected from chromium, iron, cobalt, zinc and silver) supported on a silica or a silica-magnesia carrier having an average particle diameter of 20-150µm.

US6040472A discloses novel catalyst for use in producing a carboxylic ester from an aldehyde, an alcohol and molecular oxygen, which comprises a carrier having_supported thereon Pd and Pb in a Pd/Pb atomic ratio (S) of 3/0.7</=S</=3/1.3, and which exhibits a maximum intensity peak at a diffraction angle (2 theta) in the range of from 38.55 DEG to 38.70 DEG in a powder X-ray diffraction pattern thereof, wherein the peak is ascribed to the (111) crystal face diffraction of a Pd-Pb intermetallic compound.

GB2094782A discloses a process for the production of alkyl (especially methyl) methacrylates from propylene is provided which involves fewer steps than hitherto.

In view of the deficiencies of the prior art, it would be desirable to have an improved oxidative esterification catalyst for selectively producing MMA.

### SUMMARY OF THE INVENTION

The process of the invention is such a process for the preparation of MMA via oxidative esterification, the process comprising contacting MAL, methanol, and an oxygen-containing gas in a reaction zone in the presence of a catalyst comprising palladium, bismuth, and antimony under reaction conditions sufficient to produce MMA.

Surprisingly, the oxidative esterification process of the invention provides a high selectivity to MMA.

### DETAILED DESCRIPTION OF THE INVENTION

The process of the invention is a process for the preparation of methyl methacrylate via oxidative esterification, the process comprising contacting methacrolein, methanol, and an oxygen-containing gas in a reaction zone in the presence of a catalyst comprising palladium, bismuth, and antimony under reaction conditions sufficient to produce methyl methacrylate, wherein the catalyst is a supported catalyst and the support comprises alumina.

Methanol is widely commercially available. MAL can be produced by various industrial scale processes, as known by those skilled in the art. See, e.g., US Patents 4,329,513 and 5,969,178.

The ratio of methanol fed to the amount of MAL fed in the reaction of this invention is not particularly limited. The reaction may be conducted over a wide range of methanol to MAL molar ratios, such as 1:10 to 1,000:1, preferably from 1:2 to 50:1, more preferably from 2:1 to 15:1.

The oxygen-containing gas may be either oxygen gas or a mixed gas comprising oxygen gas and a diluent inert to the reaction such as, for example, nitrogen, carbon dioxide or the like. Air may be used as the oxygen-containing gas. The oxygen-containing gas may be enriched air having a higher oxygen concentration than air, or can be pure oxygen. The quantity of oxygen present in the reaction system advantageously is not less than the stoichiometric quantity required for the reaction, and preferably is not less than 1.2 times the stoichiometric quantity. In one embodiment of the invention, the amount of oxygen present in the reaction system is from 1.2 to 2 times the stoichiometric quantity required. Hydrogen peroxide may be introduced into the reaction system as an oxidizer. The oxygen-containing gas can be introduced to the reaction system by an suitable means, as known by those skilled in the art. For example, the oxygen-containing gas can be introduced via a sparger or a pipe into a reactor. The simple method of blowing the oxygen-containing gas into the reaction system can be employed.

The catalyst advantageously is a heterogeneous catalyst comprising a catalytic metal on a porous carrier or support, where the catalytic metal comprises palladium, bismuth, and antimony. Preferably, any catalytic metal is in the reduced state, namely zero valency, and not in the cationic state, and may be present in the reduced state or as one or more compounds. The catalytic metals are present in the reaction system in such a form that they can have some interaction with each other. For example, palladium, bismuth and antimony may form an alloy, or have some other interaction, such as an intermetallic compound. In one embodiment of the invention, the catalyst may comprise a palladium intermetallic compound in which the lattice of palladium has been replaced with a diverse metal, e.g., bismuth or antimony. In another embodiment of the invention, the catalyst may comprise a palladium alloy in which palladium and a diverse metal have formed a solid solution. The ratio of palladium to bismuth in the catalyst is preferably 20:1 to 1:10 (weight ratio), and more preferably is from 5:1 to 1:1. The ratio of Sb to bismuth is advantageously from 100:1 to 1:10 (weight ratio), and in various embodiments of the invention is from 1:1 to 1:4. In one embodiment of the invention, the catalyst and/or the catalytic metal is free of added lead.

The catalytic metals are supported on a carrier or support, comprising alumina, and the amount of the catalytic constituents supported on the carrier advantageously may be from 0.1 to 20% by weight, preferably 1 to 10% by weight, based on the weight of the carrier. Examples of carriers include delta alumina, theta alumina, alumina doped with silica (also referred to herein as alumina-silica) or other materials, and gamma alumina. The carrier may be modified, as is known by those skilled in the art. Combinations of carriers may be employed. The catalyst constituents may also be used in the metallic form or in the form of compounds without supporting them on a carrier. In one embodiment of the invention, the catalyst is homogeneous.

The catalyst can be prepared in a conventional manner. For example, a soluble salt, such as palladium chloride, can be reduced with a reducing agent, such as formalin, in aqueous solution to deposit metallic palladium and the deposited metallic palladium can be filtered to prepare a metallic palladium catalyst, or a suitable carrier can be impregnated with an aqueous acidic solution of a soluble palladium salt and the impregnated carrier subjected to reduction with a reducing agent to prepare a supported palladium catalyst. In one embodiment of the invention, when it is intended to prepare a catalyst in which palladium, bismuth and antimony, are supported on a carrier, a suitable carrier is impregnated with an aqueous solution of a soluble palladium salt, and the impregnated carrier is reduced with a suitable reducing agent, after which the reduced carrier is immersed in an aqueous solution of a bismuth compound and a compound of antimony, and then dried. Alternatively, the catalyst may be prepared by first supporting the bismuth compound on the carrier, then impregnating the carrier with palladium and at least one antimony compound, and thereafter adding a reducing agent, such as hydrazine. In one embodiment of the invention, the 3 catalytic metal compounds are all introduced prior to reduction. Thus, the metals can be added in any sequence and in any combination appropriate to produce a workable catalyst. Other examples of reducing agents include formic acid, methanol, hydrogen gas and the like.

As the bismuth compound used in the preparation of the above catalyst, any suitable bismuth-containing compound may be used. For example, fatty acid salts of bismuth, such as bismuth acetate, bismuth stearate, and the like can be employed. Other suitable compounds include bismuth oxide; bismuth hydroxide; and bismuth nitrate. These bismuth compounds may be anhydrous or may be in the form of a hydrate. As the antimony compound used in the preparation of the above catalyst, any suitable antimony-containing compound may be used. Examples of antimony-containing compounds include antimony acetate, antimony chloride, antimony nitrate, and antimony sulfate. These antimony compounds may be anhydrous or may be in the form of a hydrate.

As the palladium compound used in the preparation of the catalyst, any suitable palladium-containing compound may be used. For example, fatty acid salts of palladium, such as palladium acetate, palladium stearate, and the like can be employed. Other suitable compounds include palladium oxide; palladium hydroxide; and palladium nitrate. These palladium compounds may be anhydrous or may be in the form of a hydrate.

The surface area of the catalyst advantageously is sufficient to allow the reaction to proceed. In various embodiments of the invention, the surface area of the catalyst is at least 50 m²/g, at least 60 m²/g, at least 70 m²/g, or at least 100 m²/g. These surface areas are as measured by the Brunauer-Emmett-Teller (BET) method. The BET method is described by R. B. Anderson, Experimental Methods in Catalytic Research, pp. 48-66, Academic Press (1968). In various embodiments of the invention, the surface area of the catalyst is not more than 400 m²/g, not more than 350 m²/g, or not more than 300 m²/g.

The median particle size of the catalyst, particularly when the catalyst will be used in a slurry, advantageously is from 1 to 200 microns, preferably is from 3 to 120 microns, and more preferably 5 to 100 microns. The median particle size is volume based, and is a D₅₀ size in microns that splits the particle size distribution, with half the observed volume above, and half below, that diameter. In various embodiments of the invention, when the catalyst will be used in a fixed bed, the median particle size advantageously will be larger such as, for example, from 1 to 10 mm.

It is possible to employ as the catalyst an article that has a supported catalytic metal layer in a specific shallow region in the vicinity of the outer surface of the carrier and that further has a layer on the outer surface of the carrier that is substantially free of catalytic metal. Catalysts of this type are described in US 6,228,800, the teachings of which are incorporated herein by reference. The catalyst can also have the catalytic metal distributed evenly on the surface of the carrier or can have the catalytic metal distributed evenly or homogeneously throughout the carrier.

The catalyst may be subjected to activation and/or regeneration, as is known to those skilled in the art. For example, US 6,040,472 discloses various catalyst activation techniques.

The catalyst is employed in a catalytic amount. The amount of the catalyst, i.e., catalytic metals and optional carrier, may be varied freely depending on the kind and amount of the starting materials, the method of preparing the catalyst, composition of the catalyst, process operating conditions, reactor type, and the like, although the weight ratio of catalyst to the starting aldehyde generally is from 1:1000 to 20:1. Advantageously, the ratio of catalyst to aldehyde is from 1:100 to 4:1. However, the catalyst may be used in an amount outside these ranges.

The process for producing MMA comprises contacting reactants comprising MAL, methanol and an oxygen-containing gas, under oxidative esterification conditions in the presence of the catalyst. Oxidative esterification conditions include, for example, the oxygen partial pressure, reaction total pressure, temperature, concentration of reactants, pH and reaction time suitable to produce the desired reaction product. In one embodiment of the invention, the reaction may be conducted using a slurry of the catalyst in the liquid phase in the reaction zone. The reaction may be conducted at a temperature of from 0°C to 120°C, preferably from 40°C to 90°C. The reaction may be conducted at reduced pressure, at atmospheric pressure, or at superatmospheric pressure. The reaction pressure for oxidative esterification reactions is advantageously selected within a range at which the catalyst is active for oxidative esterification reactions. The reaction may be conducted at a pressure of from 50 to 2000 kPa (7.3 to 290 psia), preferably from 100 to 1000 kPa (14.5 to 145 psia). The reaction may be conducted in a batch, semi-batch or continuous manner.

The reaction may be conducted in any suitable reactor type such as, for example, a CSTR, a bubble column reactor or a fixed bed reactor. The reactor can be stirred or not stirred, and may have a mobile catalyst that generally moves with the reaction liquid, or may contain a fixed bed of catalyst through which the reaction fluid flows. Recycling of reaction fluids through the reactor can be conducted in any of these configurations. In an embodiment, a single reactor with a single reaction zone is used.

In one embodiment of the invention, the reaction is carried out in the slurry phase. The catalyst may then be separated from the product mixture, for example, by filtration or decantation. In various embodiments of the invention, the "reaction fluid," which may be a mixed phase composition comprising solids, liquids and gases, may contain a heterogeneous catalyst, e.g., slurry, or at least a portion of the reaction fluid may contact a fixed bed of catalyst during the process.

In an embodiment, the oxygen partial pressure varies depending on the reactants, reaction conditions and type of reactor. In an embodiment, the oxygen partial pressure on the outlet side of the reactor is a positive pressure of less than or equal to 35 kPa (5 psia). In an embodiment, the oxygen partial pressure on the outlet side of the reactor is a positive pressure of less than or equal to 200 kPa (29 psia).

In an embodiment, the pH of the reaction is maintained in the range of 6 to 9. If necessary to maintain the pH, an alkaline material, such as an alkali metal compound or alkaline earth metal compound, may be added to the reaction. Exemplary alkaline materials include alkali metal and alkaline earth metal compounds that include, but are not limited to, oxides, hydroxides, carbonates, and carboxylic acid salts.

The reaction time varies depending on the reaction conditions, reactants and other factors which may influence the reaction. Typically, however, the reaction time is from 0.5 to 20 hours. For a continuous process, such as in embodiments using a continuous stirred tank reactor CSTR, the reaction time (residence time) is governed by the kinetics of the system as determined by the pressure, temperature and catalyst used.

In an embodiment, the process includes removing a crude product stream from the reactor. The crude product stream comprises MMA along with unreacted methanol, MAL and oxygen, as well as various amounts of by-products such as, for example, water, methacrylic acid, methyl formate and other by-products.

A polymerization inhibitor can be employed in the process when the product is a polymerizable compound. A wide variety of inhibitors are known and commercially available. Examples of inhibitors include hydroquinone (HQ), phenothiazine (PTZ), the methyl ester of hydroquinone (MEHQ), 4-hydroxy-2 2 6 6-tetramethylpiperidine-n-oxyl (4-hydroxy TEMPO, or 4HT), methylene blue, alkyl-aryl-phenylenediamine, copper salicylate, copper dialkyldithiocarbamates, and the like.

In various embodiments of the invention, the catalyst is employed in said esterification to provide a selectivity to MMA of at least 90%, or at least 95%, or at least 98%, or at least 99%, based on MAL. For the purposes of the invention, yield is calculated as the mathematical product of conversion times selectivity.

### SPECIFIC EMBODIMENTS OF THE INVENTION

The following examples are given to illustrate the invention.

### Calculation of Conversion and Selectivity:

Conversion and selectivity are calculated ignoring a 5 hour catalyst activation period. The concentration of various constituents is obtained at the fifth hour of operation and the twenty-second hour of operation. Condensate from the dry ice condenser is returned to the reactor, and the samples are dilute in nature. Organic vapor losses and changes to sample weights are assumed to be minimal. The reactor contents are analyzed via a gas chromatograph (GC) with a flame ionization detector (FID).

MAL conversion is calculated as the moles of MAL reacted during the relevant time period (i.e., the moles of MAL present at the fifth hour minus the moles of MAL present at the twenty second hour) divided by the moles of MAL present at the fifth hour, and is expressed as a percentage.

Selectivity to MMA is calculated as the moles of MMA made (from hour five to hour twenty two) divided by the moles of MAL consumed over that time period, and is also expressed as a percentage.

### Example 1

Catalyst Preparation: A catalyst having 5 wt% Pd, 2 wt% Bi, and 1 wt% Sb on an alumina carrier is prepared using Sigma Aldrich 5 wt% Pd on alumina as a starting point. A slurry is prepared by dissolving 0.90 grams of bismuth nitrate pentahydrate in 100 ml of deionized water, then adding 0.47 g antimony acetate to provide 1 wt% Sb on a carrier basis, and then adding 20.0 grams of the Aldrich Pd/alumina. The slurry is stirred for 1 hour at 60°C, then 10.0 grams of hydrazine hydrate are added slowly, dropwise, and the resulting mixture is stirred for an additional 1 hour at 90°C. The resulting solids are then separated via vacuum filtration, are washed with 500 ml of deionized water, and are vacuum dried at 45°C for 10 hours.

MMA Preparation: A 5 gram sample of the catalyst is placed in a 300 ml glass reactor with a 100 g solution of 4.0 wt% MAL in methanol. The glass reactor is fitted with a pitched-blade turbine impeller, dry ice condenser and dry ice traps. The reactor is maintained at 40°C by immersion in a constant temperature bath and is operated at atmospheric pressure. The reactor is run in a semi-batch mode having a batch liquid phase organic reactant and catalyst slurry that is continuously sparged by 8% O₂ in N₂ gas. The gas flows continuously into the liquid through a fine glass frit which generates small bubbles. The bubbles and the catalyst are well distributed throughout the liquid due to agitation induced by the impeller. The solution also contains, as a polymerization inhibitor, approximately 50 ppm 4-HT in combination with PTZ (approximately 10 ppm) and HQ (approximately 10 ppm). The reactor is operated in this manner for a total of approximately 22 hours and samples are obtained at the beginning of the experiment, at the fifth hour of the experiment, and at the end of the experiment.

Conversion of MAL is 100%. Selectivity to MMA is approximately 86% based on MAL. The yield is calculated as 1 x 0.86 = 86%.

### Example 2

Example 1 is repeated except that the solution of MAL in methanol contains 4.7 wt% MAL.

Conversion of MAL is 92%. Selectivity to MMA is above 99% based on MAL. The yield is calculated as 0.92 x 0.99 = 91%.

### Example 3

Catalyst Preparation: A catalyst having 5 wt% Pd, 2 wt% Bi, and 1 wt% Sb on a gamma alumina carrier is prepared using 5 wt% Pd and 2 wt% Bi on alumina as a starting point. The carrier is T-2610 micro-spherical gamma alumina from Clariant, having a median particle size of from 55 to 80 microns and a surface area of from 120 to 150 m²/g. This catalyst is made by first using incipient wetness impregnation of the nitrate salt of Pd followed by calcining in air at atmospheric pressure and for sufficient time to denitrify the material. Bi is then added to the material by incipient wetness impregnation of the nitrate salt of Bi followed by calcining in air at atmospheric pressure for sufficient time to denitrify that material. A slurry is prepared by dissolving 0.47 g antimony acetate to provide 1 wt% Sb on a carrier basis, and then adding 20.0 grams of the Pd/Bi material. The slurry is stirred for 1 hour at 60°C, after which 10.0 grams of hydrazine hydrate are added slowly, dropwise, and stirred for an additional 1 hour at 90°C. The resulting solids are then separated via vacuum filtration, are washed with 500 ml of deionized water, and are vacuum dried at 45°C for 10 hours.

MMA Preparation: A 5 gram sample of the catalyst is placed in a glass reactor with a 100 g solution of 4.4 wt% MAL in methanol. The solution also contains, as polymerization inhibitor, approximately 50 ppm 4-HT in combination with PTZ (approximately 10 ppm) and HQ (approximately 10 ppm).

Conversion of MAL is 100%. Selectivity to MMA is approximately 82% based on MAL. The yield is calculated as 1 x 0.82 = 82%.

### Example 4

MMA Preparation: Example 3 is repeated except that the solution of MAL in methanol contains 4.5 wt% MAL.

Conversion of MAL is 100%. Selectivity to MMA is approximately 93% based on MAL. The yield is calculated as 1 x 0.93 = 93%.

### Example 5

Catalyst Preparation: A catalyst having 5 wt% Pd, 2 wt% Bi, and 1 wt% Sb on a silica-alumina carrier is prepared using 5 wt% Pd and 2 wt% Bi on silica-alumina as a starting point. The carrier is T-2865 micro-spherical silica-modified gamma alumina from Clariant, having a median particle size of from 55 to 80 microns and a surface area of from 120 to 150 m²/g. This catalyst is made by first using incipient wetness impregnation of the nitrate salt of Pd followed by calcining in air at atmospheric pressure and for sufficient time to denitrify the material. Bi is then added to the material by incipient wetness impregnation of the nitrate salt of Bi followed by calcining in air at atmospheric pressure for sufficient time to denitrify that material. A slurry is prepared by dissolving 0.47 g antimony acetate to provide 1 wt% Sb on a carrier basis, and then adding 20.0 grams of the Pd/Bi material. The slurry is stirred for 1 hour at 60°C, after which 10.0 grams of hydrazine hydrate are added slowly, dropwise, and stirred for an additional 1 hour at 90°C. The resulting solids are then separated via vacuum filtration, are washed with 500 ml of deionized water, and are vacuum dried at 45°C for 10 hours.

MMA Preparation: Example 3 is repeated except that the solution of MAL in methanol contains 4.4 wt% MAL.

Conversion of MAL is 100%. Selectivity to MMA is above 99% based on MAL. The yield is above 99%.

### Example 6

MMA Preparation: Example 5 is repeated.

Conversion of MAL is 100%. Selectivity to MMA is above 99% based on MAL. The yield is above 99%.

## Claims

1. A process for the preparation of methyl methacrylate via oxidative esterification, the process comprising contacting methacrolein, methanol, and an oxygen-containing gas in a reaction zone in the presence of a catalyst comprising palladium, bismuth, and antimony under reaction conditions sufficient to produce methyl methacrylate, wherein the catalyst is a supported catalyst and the support comprises alumina.

2. The process of any of the preceding claims wherein the weight ratio of palladium to bismuth in the catalyst is from 20:1 to 1:10.

3. The process of any of the preceding claims wherein the weight ratio of antimony to bismuth is from 100:1 to 1:10.

4. The process of any of the preceding claims wherein the amount of the catalytic metal on the support is from 0.1 to 20% by weight, based on the weight of the support.

5. The process of any of the preceding claims wherein the surface area of the catalyst is at least 50 m²/g.

6. The process of any of the preceding claims wherein the median particle size of the supported catalyst is from 1 to 200 microns.

7. The process of any of the preceding claims wherein the weight ratio of palladium to bismuth in the catalyst is from 5:1 to 1:1, and the weight ratio of Sb to bismuth is from 1:1 to 1:4.

8. The process of any of the preceding claims wherein the weight ratio of the catalyst in the reaction zone to methacrolein fed to the reaction zone is from 1:1000 to 20:1.

9. The process of any of the preceding claims wherein the temperature in the reaction zone is from 0°C to 120°C, and the pressure in the reaction zone is from 50 to 2000 kPa (7.3 to 290 psia).

## Patentansprüche

1. Ein Verfahren zur Herstellung von Methylmethacrylat durch oxidative Veresterung, wobei das Verfahren das In-Kontakt-Bringen von Methacrolein, Methanol und einem sauerstoffhaltigen Gas in einer Reaktionszone in Gegenwart eines Katalysators beinhaltet, der Palladium, Bismut und Antimon unter ausreichenden Reaktionsbedingungen, um Methylmethacrylat zuzubereiten, beinhaltet, wobei der Katalysator ein Trägerkatalysator ist und der Träger Aluminiumoxid beinhaltet.

2. Verfahren gemäß einem der vorhergehenden Ansprüche, wobei das Gewichtsverhältnis von Palladium zu Bismut in dem Katalysator von 20 : 1 bis 1 : 10 ist.

3. Verfahren gemäß einem der vorhergehenden Ansprüche, wobei das Gewichtsverhältnis von Antimon zu Bismut von 100 : 1 bis 1 : 10 ist.

4. Verfahren gemäß einem der vorhergehenden Ansprüche, wobei die Menge des katalytischen Metalls auf dem Träger von 0,1 bis 20 Gew.-%, bezogen auf das Gewicht des Trägers, ist.

5. Verfahren gemäß einem der vorhergehenden Ansprüche, wobei der Oberflächenbereich des Katalysators mindestens 50 m²/g ist.

6. Verfahren gemäß einem der vorhergehenden Ansprüche, wobei die mittlere Teilchengröße des Trägerkatalysators von 1 bis 200 Mikrometer ist.

7. Verfahren gemäß einem der vorhergehenden Ansprüche, wobei das Gewichtsverhältnis von Palladium zu Bismut in dem Katalysator von 5 : 1 bis 1 : 1 ist und das Gewichtsverhältnis von Sb zu Bismut von 1 : 1 bis 1 : 4 ist.

8. Verfahren gemäß einem der vorhergehenden Ansprüche, wobei das Gewichtsverhältnis des Katalysators in der Reaktionszone zu Methacrolein, das der Reaktionszone zugeführt wurde, von 1 : 1000 bis 20 : 1 ist.

9. Verfahren gemäß einem der vorhergehenden Ansprüche, wobei die Temperatur in der Reaktionszone von 0 °C bis 120 °C ist und der Druck in der Reaktionszone von 50 bis 2000 kPa (7,3 bis 290 psia) ist.

## Revendications

1. Un procédé pour la préparation de méthacrylate de méthyle via une estérification oxydante, le procédé comprenant la mise en contact de méthacroléine, méthanol, et un gaz contenant de l'oxygène dans une zone de réaction en présence d'un catalyseur comprenant du palladium, du bismuth, et de l'antimoine sous des conditions de réaction suffisantes pour produire du méthacrylate de méthyle, dans lequel le catalyseur est un catalyseur supporté et le support comprend de l'oxyde d'aluminium.

2. Le procédé de n'importe lesquelles des revendications précédentes dans lequel le rapport en poids du palladium au bismuth dans le catalyseur est de 20/1 à 1/10.

3. Le procédé de n'importe lesquelles des revendications précédentes dans lequel le rapport en poids de l'antimoine au bismuth est de 100/1 à 1/10.

4. Le procédé de n'importe lesquelles des revendications précédentes dans lequel la quantité du métal catalytique sur le support est de 0,1 à 20 % en poids, rapporté au poids du support.

5. Le procédé de n'importe lesquelles des revendications précédentes dans lequel la surface spécifique du catalyseur est au moins de 50 m²/g.

6. Le procédé de n'importe lesquelles des revendications précédentes dans lequel la taille de particules médiane du catalyseur supporté est de 1 à 200 micromètres.

7. Le procédé de n'importe lesquelles des revendications précédentes dans lequel le rapport en poids du palladium au bismuth dans le catalyseur est de 5/1 à 1/1, et le rapport en poids du Sb au bismuth est de 1/1 à 1/4.

8. Le procédé de n'importe lesquelles des revendications précédentes dans lequel le rapport en poids du catalyseur dans la zone de réaction à la méthacroléine introduite dans la zone de réaction est de 1/1 000 à 20/1.

9. Le procédé de n'importe lesquelles des revendications précédentes dans lequel la température dans la zone de réaction est de 0 °C à 120 °C, et la pression dans la zone de réaction est de 50 à 2 000 kPa (7,3 à 290 psia).
